Europäisches Patentamt

European Patent Office

Office européen des brevets

(19)

(11) Numéro de publication : **0 179 703**
**B1**

(12) **FASCICULE DE BREVET EUROPÉEN**

(45) Date de publication du fascicule du brevet :
**25.05.88**

(21) Numéro de dépôt : **85401934.6**

(22) Date de dépôt : **03.10.85**

(51) Int. Cl.⁴ : **C 07 C 31/26, B 01 J 2/12,
B 01 J 2/14, A 61 K 9/20**

(54) **Mannitol granulaire directement compressible et son procédé de fabrication.**

(30) Priorité : **03.10.84 FR 8415211**

(43) Date de publication de la demande :
**30.04.86 Bulletin 86/18**

(45) Mention de la délivrance du brevet :
**25.05.88 Bulletin 88/21**

(84) Etats contractants désignés :
**AT BE CH DE GB IT LI LU NL SE**

(56) Documents cités :
**FR-A- 1 506 334**
**FR-A- 2 451 357**
**US-A- 3 341 415**

(73) Titulaire : **Roquette Frères**

**F-62136 Lestrem (FR)**

(72) Inventeur : **Serpelloni, Michel**
**270, Boulevard Voltaire**
**F-62400 Bethune (FR)**
Inventeur : **Lemay, Patrick**
**29, rue du Puits Richebourg**
**F-62136 Lestrem (FR)**

(74) Mandataire : **Koch, Gustave et al**
**Cabinet PLASSERAUD 84, rue d'Amsterdam**
**F-75009 Paris (FR)**

EP 0 179 703 B1

Jouve, 18, rue St-Denis, 75001 Paris, France

## Description

L'invention a pour objet un mannitol granulaire directement compressible.

Elle vise également un procédé de fabrication de ce mannitol.

Le mannitol, de par sa saveur et son hygroscopicité faible, constitue un excipient pharmaceutique de choix dès lors qu'on le rend directement compressible ; or il ne présente pas naturellement cette propriété, notamment lorsqu'il est obtenu par cristallisation dans l'eau ; il est, par ailleurs, naturellement très friable et subit par conséquent un effritement important lors des transports ; un tel effritement est générateur de fines particules qui ont un effet néfaste sur l'aptitude à l'écoulement et sur la compressibilité.

Il s'ensuit que les propriétés du mannitol granulaire directement compressible que l'on trouve dans le commerce ne sont pas satisfaisantes.

Dans ce cas, la friabilité et la compressibilité sont très éloignées de celles de l'excipient pharmaceutique que l'on prend habituellement comme référence, à savoir le lactose qui peut être directement compressible mais qui présente précisément des défauts dont la mannitol est exempt, à savoir :

— un trop grande hygroscopicité,

— un pouvoir réducteur,

— une trop grande cariogénéicité.

Il a déjà été tenté de remédier à ces inconvénients en incorporant au mannitol un liant.

Or, la présence d'un liant n'est pas très prisée par l'utilisateur.

Il a également été proposé (voir à cet égard le brevet US-A-3 341 415) d'améliorer les caractéristiques physiques du mannitol en le mettant en œuvre en combinaison avec certains sucres, tels que le lactose ou le sucrose, notamment sous la forme de solutions solides avec ces derniers, mais alors on retrouve les inconvénients précités.

Il existait, par conséquent, un besoin en un mannitol granulaire très peu friable, donc facilement transportable, et directement compressible, ne comportant pas de liant et égalant, voire surclassant le lactose granulaire sans liant de granulation.

C'est ce besoin que la Société Demanderesse s'est efforcé de satisfaire.

Or, elle a eu le mérite de trouver qu'il était possible de préparer un mannitol granulaire directement compressible sans liant, d'une friabilité substantiellement plus faible que celle de tous les mannitols directement compressibles connus, y compris ceux comportant un liant.

Ce mannitol directement compressible et sans liant est un produit industriel nouveau et constitue l'objet principal de l'invention.

Suivant un aspect de l'invention, le susdit mannitol granulaire, directement compressible et exempt de liant, est caractérisé par le fait qu'il présente une friabilité inférieure à 65 % dans un test A.

Ce mannitol présente, simultanément à cette faible friabilité, une compressibilité supérieure à 49,0 N, de préférence à 78,4 N dans un test B.

De plus, il présente un excellent indice d'écoulement, supérieur à 65 dans un test C.

La friabilité traduit la résistance à un concassage des granules de mannitol.

Elle est illustrée par la proportion de granules qui, après le concassage, n'est pas retenue par un tamis de largeur de mailles donnée, c'est-à-dire a subi une diminution des dimensions des particules telle que celles-ci puissent passer par les mailles dudit tamis.

Pour apprécier la friabilité, on peut avoir recours à un test A.

Celui-ci consiste à soumettre les granules à tester à un concassage effectué dans un appareil dénommé friabilimètre : dans le test A, on utilise le friabilimètre de marque « ERWEKA TAP » fabriqué par la Société ERWEKA (6056 Heusenstamm R.F.A.), tournant à une vitesse de rotation uniforme de 25 tours/minute, et dans lequel on a introduit 5 billes d'acier identiques d'un diamètre de 17 mm et d'un poids de 18,87 g.

On introduit dans la chambre de concassage du friabilimètre une quantité de 15 g de granules correspondant à la fraction granulométrique de 400 à 500 $\mu$m ; on met l'appareil en rotation pendant 15 minutes.

A la fin de l'expérience, on détermine la proportion pondérale représentée par le résidu retenu sur un tamis d'une largeur de maille de 351 microns.

La valeur de la friabilité correspond au pourcentage de poudre non retenu par le tamis précédemment défini.

La friabilité est d'autant plus faible que le pourcentage de poudre non retenu par le susdit tamis est faible.

Pour la mesure de la compressibilité, on peut avoir recours au test B qui consiste à fabriquer, à l'aide d'une presse rotative à haut rendement, des tablettes et à en mesurer la résistance à la rupture.

La presse utilisée est une presse de type P 1000 fabriquée par la Société WILHELM FETTE (2053 Schwarzenbeck R.F.A.) ; elles est équipée de jauges de mesure des forces de compression et d'éjection.

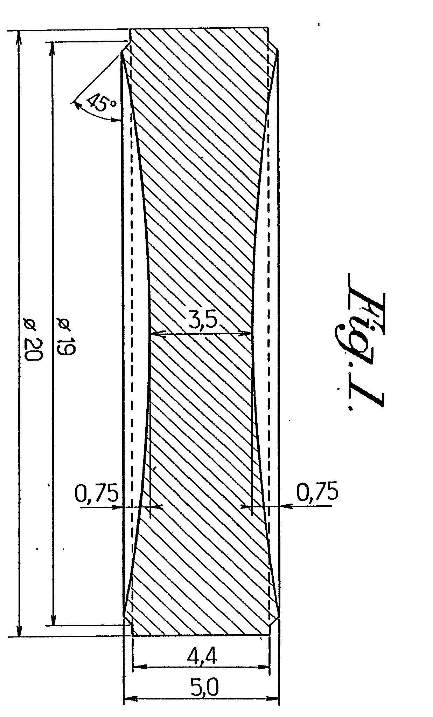

Les tablettes préparées sont rondes, biconcaves de 20 mm de diamètre et de 5 mm d'épaisseur ; leur forme et leurs dimensions sont celles apparaissant sur la figure 1, qui est une coupe diamétrale d'un comprimé préparé à partir d'une poudre du produit à tester.

La pression de compression appliquée est de 196,133 MPa et le mannitol poudre utilisé est additionné d'un lubrifiant, en l'occurrence le stéarate de magnésium, en une quantité telle que la force d'éjection reste inférieure à 500 newtons (N), conditions observées de bon fonctionnement de l'appareillage.

Là compressibilité, exprimée par la valeur de la résistance à la rupture, en newton, est mesurée à l'aide d'un duromètre ERWEKA TB 24, équipé d'un chevalet de support de tablettes, ayant une distance entre appuis de 10,55 mm.

Pour la mesure de l'indice d'écoulement, on peut avoir recours à un test C qui fournit en fait ce qui s'appelle l'indice de CARR ; le test C est décrit dans Chem. Eng. 72, N° 1, 163-168 (1965) et Chem. Eng. 72, N° 2, 69-73 (1965).

On peut utiliser à cet égard l'appareil commercialisé par la Société HOSOKAWA sous la marque « Powder Tester ».

Dans les exemples qui seront décrits plus loin, on fournit, pour les produits granulaires directement compressibles testés (mannitol conforme à l'invention, mannitol de l'art antérieur, lactose) également d'autres grandeurs, à savoir :
— la répartition granulométrique, et la granulométrie moyenne,
— la valeur de la surface spécifique,
— la valeur de la porosité.

La granulométrie moyenne (taille moyenne correspondant à 50 % de la distribution en masse) présentée par le mannitol conforme à l'invention peut être inférieure à 650 μm, favorisant ainsi son utilisation dans la préparation de comprimés pharmaceutiques faiblement dosés en principes actifs. La friabilité traduit la résistance à un concassage des granules de mannitol.

La « surface spécifique » est exprimée en m² par g de produit. Elle est mesurée par la méthode bien connue du BET (voir Techniques de l'Ingénieur, 1968, vol. 5, p. 3645, lignes 1-11).

La porosité est exprimée en cm³/g. Elle est mesurée par porosimétrie au mercure (voir op. cit. Techniques de l'Ingénieur).

Pour préparer le mannitol granulaire directement compressible et exempt de liant conforme à l'invention, on peut avoir recours, toujours conformément à l'invention, à un procédé adapté à partir de celui décrit dans le brevet FR-A-2 451 357 en rapport avec la préparation de sorbitol cristallisé et caractérisé par le fait que l'on introduit simultanément et en continu dans un récipient ouvert, d'axe horizontal ou incliné sur l'horizontale, entraîné en rotation autour dudit axe à une vitesse de 2 à 10 tours/minute, contenant une masse de granules de mannitol d'une température de 145 à 165 °C, maintenue en mouvement par la rotation du récipient,
— d'une part, du mannitol cristallisé d'une granulométrie inférieure à 5 mm, de préférence inférieure à 1,5 mm, et d'une température de 5 à 150 °C, de préférence de 50 à 150 °C,
— d'autre part, du mannitol essentiellement à l'état liquide, divisé ou non en gouttelettes d'une teneur en matières sèches supérieure à 99 % et d'une température comprise entre 164 et 180 °C et, de préférence de 166,5 à 180 °C, et plus avantageusement encore de 175 à 180 °C.

Le mannitol cristallisé et le mannitol liquide étant amenés à la surface de la masse de granules en mouvement suivant un rapport de 6 à 1,5 parties en poids de mannitol liquide pour 1 partie en poids de mannitol cristallisé, ces paramètres étant sélectionnés à l'intérieur des limites indiquées de telle sorte qu'il ne se produise pas de fusion totale à la surface de la masse en mouvement où se rejoignent le mannitol essentiellement à l'état liquide et le mannitol cristallisé, les granules de grande taille qui ont tendance à monter à la surface de la masse en mouvement par un phénomène de ségrégation, étant recueillis par exemple par débordement vers l'extérieur du récipient rotatif.

De préférence, ces granules sont soumis à une phase de mûrissement par maintien à une température de 70 à 80 °C pendant 5 à 60 minutes.

Se proposant, par conséquent, de frabriquer le mannitol granulaire, directement compressible, conforme à l'invention, on s'y prend comme suit ou de façon équivalente.

Les matières premières utilisées dans le cadre du procédé selon l'invention comprennent :
— du mannitol essentiellement à l'état liquide maintenu à une température d'environ 164 à 180 °C, de préférence de 166,5 à 180 °C, et plus avantageusement encore de 175 à 180 °C, et
— du mannitol cristallisé d'une granulométrie inférieure à 5 mm et, de préférence à 1,5 mm et d'une température d'environ 5 à 150 °C, de préférence de 50 à 150 °C.

Ces deux constituants, à savoir, le mannitol liquide et le mannitol cristallisé sont introduits en continu en des proportions respectives d'environ 6 à 1,5 parties en poids pour le premier et d'environ 1 partie en poids pour le second, dans un récipient ouvert d'axe horizontal ou incliné sur l'horizontale entraîné en mouvement autour dudit axe à une vitesse de 2 à 10 tours/minute et contenant une masse de granules de mannitol maintenue en mouvement par la rotation du récipient et à une température de 145 à 165 °C par des moyens appropriés, le mélange du mannitol liquide et du mannitol cristallisé se faisant à la surface de la masse en mouvement ; ce mouvement rappelle celui d'une masse de dragées à l'intérieur d'une drageuse et c'est grâce à lui qu'il se forme des granules de taille de plus en plus grande, les granules de plus grande taille ayant tendance à venir à la surface de la masse en mouvement.

Le mannitol liquide qui est préparé par exemple par fusion de mannitol poudre dans une installation

3

agencée de façon telle que toute surchauffe soit évitée, est amené à la surface de cette masse en mouvement à l'état de faisceaux, de nappes, de filets et préférentiellement à l'état finement divisé sous forme de gouttelettes à l'aide, par exemple, de moyens de pulvérisation.

L'ensemble des paramètres est choisi de façon telle qu'à l'intérieur du récipient et à la surface de la masse en mouvement, une fusion totale des granules constitutifs de la masse ne puisse se produire.

Ces granules recueillis par débordement sont constitués par un agglomérat de mannitol fondu et de poudre cristalline de mannitol ; ils sont, de préférence, soumis à un traitement de mûrissement pendant environ 5 à 60 minutes à une température d'environ 70 à 80 °C.

Ils sont ensuite broyés, par exemple à l'aide d'un broyeur du type à marteaux.

Les particules de granulométrie inférieure à environ 5 mm et, de préférence, inférieure à 1,5 mm, sont généralement recyclées et constituent une partie de la matière première cristallisée mise en œuvre.

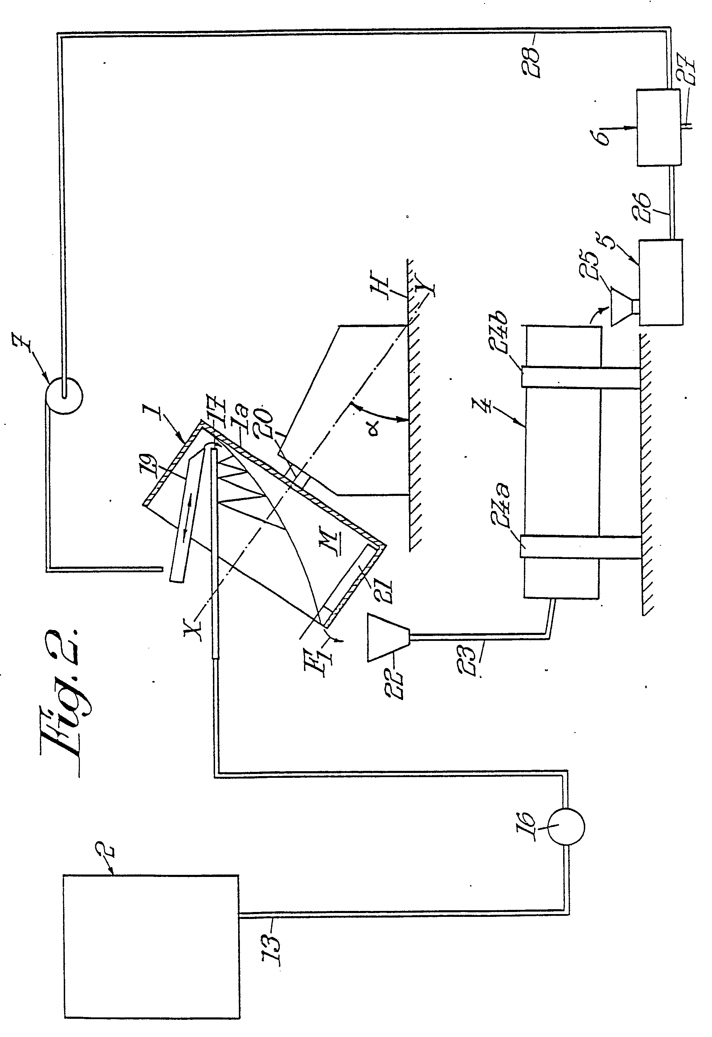

L'installation pour la mise en œuvre du procédé qui vient d'être décrit comprend essentiellement, comme il résulte de la coupe schématique montrée à la figure 2, un récipient rotatif désigné dans son ensemble par 1 et dont l'axe XY présente éventuellement une inclinaison sur l'horizontale ; ce récipient est placé en série, comme montré à la figure 1 avec, en amont :

— un dispositif d'alimentation 2 en mannitol à l'état essentiellement liquide,

— des moyens de dispersion du mannitol liquide à l'intérieur du récipient 1 et des moyens propres à mélanger au mannitol fondu, dispersé, du mannitol cristallisé de granulométrie inférieure à 5 mm et, en aval du récipient 1 :

— de préférence, une enceinte rotative 4 de « mûrissement »,

— une installation de broyage 5,

— une installation de tamisage 6,

— des moyens 7 propres à recycler à la sortie de l'installation de tamisage et vers les moyens de mélange coopérant avec le récipient 1, la partie du mannitol cristallisé broyé de fine granulométrie.

Le dispositif 2 est relié au récipient rotatif 1 par une canalisation 13· munie d'une pompe 16.

Les moyens préférés de dispersion du mannitol liquide comprennent la pompe 16 amenant au niveau du récipient 1 le mannitol liquide et le pulvérisant à l'aide d'une buse 17, par exemple, en forme de rampe, placée au voisinage du fond 1a du récipient. Les gouttelettes du mannitol liquide pulvérisé sont mises en présence de mannitol cristallisé pulvérulent amené par exemple à l'aide d'un dispositif 19 du type distributeur vibrant.

Le mélange s'effectue à la surface de la masse M en mouvement qui remplit partiellement le récipient comme montré.

La rotation de ce dernier est assurée par exemple par un dispositif à entraînement schématiquement représenté en 20.

Le récipient 1 peut être établi sous la forme d'un bac ou tambour, comme montré à la figure 1.

On peut également l'établir de façon analogue à une drageuse, c'est-à-dire sous la forme d'une sphère à laquelle on aura enlevé une calotte.

Lorsque le récipient 1 a une forme analogue à celle représentée à la figure 1, on l'utilise avec un angle d'inclinaison généralement supérieur à 25° et, de préférence, compris entre 25 et 45°.

Des moyens non représentés sont alors prévus pour faire varier ladite inclinaison de l'axe de rotation XY sur le plan horizontal.

Lorsque le récipient 1 présente une forme de drageuse, l'inclinaison peut être inférieure à 25° et même devenir nulle.

Avantageusement, le récipient 1 est équipé d'un couteau racleur 21.

Les granules qui sont amenés à la surface de la masse en mouvement M sont évacués du récipient 1 par exemple suivant un système de trop-plein (voir la flèche F₁) et acheminés ainsi à une goulotte 22 qui est reliée par une canalisation 23 à l'entrée du cylindre rotatif 4 avantageusement comporté par l'installation. Ce cylindre rotatif 4 est supporté par deux paliers 24a et 24b comportant des moyens propres à l'entraîner en rotation. Les dimensions de ce cylindre, sa vitesse de rotation et son inclinaison sont choisies de façon telle que la durée de séjour d'un granule donné à l'intérieur de ce cylindre soit comprise de préférence entre 5 et 60 minutes.

A la sortie du cylindre rotatif 4, les granules mûris et, par conséquent, constitués par du mannitol cristallisé, tombent dans une goulotte 25 et sont introduits dans la susdite installation de broyage 5. Cette installation est, de préférence, agencée de façon telle que l'on puisse faire varier la granulométrie du produit broyé obtenu.

A la sortie de cette installation de broyage, le produit broyé est acheminé par une canalisation 26, équipée de préférence de moyens d'acheminement pneumatiques, jusqu'à l'installation de tamisage 6 à la sortie de laquelle on recueille, d'une part, le produit présentant la granulométrie désirée qui est évacué par une canalisation 27 et, d'autre part, du produit de granulométrie trop faible qui est recyclé par une canalisation 28 vers le récipient 1, ladite canalisation 28 comportant des moyens 7 susmentionnés qui sont constitués, par exemple, par une turbine.

Ceci étant, pour fixer les idées, on signale que, dans une installation donnant de bons résultats et qui a été expérimentée par la Société Demanderesse, on utilise un récipient 1 en forme de bac du type de celui représenté à la figure 1, d'un diamètre de 3,60 m et d'une profondeur de 1,20 m et dont l'inclinaison est réglable entre 25 et 45° ; on confère en général à ce bac une vitesse de rotation d'environ 7 tours/minute. Dans cette même installation, on utilise un cylindre 4 dont la longueur est de 8,50 m, le diamètre de 1,80 m, l'inclinaison de 5° et la vitesse de rotation de 10 t/mn.

Pour illustrer ce qui précède, on donne ci-après des exemples de préparation de mannitol granulaire directement compressible conforme à l'invention par mise en œuvre du procédé et de l'installation conformes à l'invention.

## Exemple 1

On procède à l'alimentation de l'installation décrite à raison de 375 kg/heure en mannitol poudre de granulométrie moyenne de 120 microns et de 800 kg/heure de mannitol fondu, à 175 °C, et on règle l'inclinaison du récipient 1 à une valeur de 30°.

La température régnant au sein de la masse de granules en mouvement est de 160 à 165 °C.

Le temps de séjour moyen dans l'enceinte de mûrissement est de 25 minutes.

La taille moyenne des granules récupérés par débordement est de 5 à 15 mm.

Le mannitol granulaire directement compressible ainsi obtenu présente les caractéristiques suivantes :

| | |
|---|---|
| — répartition granulométrique | $> 1\ 000\ \mu m : 0,5\ \%$ |
| | $> 500\ \mu m : 48\ \%$ |
| | $> 250\ \mu m : 99\ \%$ |
| — granulométrie moyenne | : 520 micron |
| — résistance à la rupture (exprimé en newton, appareil « ERWEKA ») | : 97,0 |
| — friabilité | : 45 % |
| — écoulement | : 85 |
| — surface spécifique | : < 0,4 m²/g |
| — porosité en cm³/g | : < 1 |

## Exemple 2

On procède comme dans l'exemple 1 mais en modifiant les paramètres suivants : l'inclinaison du récipient 1 passe à une valeur de 35°.

Les granules récupérés par débordement présentent une granulométrie moyenne de 5 à 10 mm et sont directement soumis au broyage.

Le mannitol granulaire directement compressible ainsi obtenu présente les caractéristiques suivantes :

| | |
|---|---|
| — répartition granulométrique | $> 1\ 000\ \mu m : 3\ \%$ |
| | $> 500\ \mu m : 73\ \%$ |
| | $> 250\ \mu m : 99\ \%$ |
| — granulométrie moyenne | : 620 microns |
| — résistance à la rupture (exprimé en newton, appareil « ERWEKA ») | : 91,2 |
| — friabilité | : 45 % |
| — écoulement | : 85 |
| — surface spécifique | : < 0,4 m²/g |
| — porosité en cm³/g | : < 1 |

Dans le tableau, on a réuni les caractéristiques de granulométrie, de compressibilité, de friabilité et d'écoulement :
— des mannitols des exemples 1 et 2,
— d'un lactose directement compressible du commerce,
— d'un mannitol directement compressible et sans liant du commerce,
— d'un mannitol directement compressible comportant un liant.

La supériorité du mannitol directement compressible conforme à la présente invention apparaît clairement à l'examen des résultats réunis dans le présent tableau.

Sa friabilité présente une valeur tellement faible qu'elle n'a aucune commune mesure avec celle des mannitols directement compressibles existants, même avec liant ; le transport du mannitol granulaire directement compressible selon l'invention ne pose donc guère de problème du point de vue effritement et formation de fines particules gênantes.

Sa compressibilité est supérieure à celle du lactose.

Son indice d'écoulement, égal ou supérieur à celui du lactose et du mannitol directement compressible du commerce avec ou sans liant, est suffisamment élevé pour qu'une exploitation commerciale comme excipient de compression directe puisse être retenue.

Tableau

| | Lactose directement compressible | Mannitol selon l'exemple 1 | Mannitol selon l'exemple 2 | Mannitol directement compressible du commercè sans liant | Mannitol directement compressible avec liant (1,5% de gélatine) |
|---|---|---|---|---|---|
| Répartition granulo- ) > 1000 µm<br>métrique en % ) > 500 µm<br>cumulé en masse ) > 250 µm | 0<br>0<br>3 | 0,5<br>48<br>99 | 3<br>73<br>99 | 8,1<br>95<br>99 | 3<br>77<br>99 |
| Granulométrie moyenne (en µm) * | 90 | 520 | 620 | 860 | 650 |
| Teneur en % de stéarate de magnésium | 1 | 0,5 | 0,5 | 0,8 | 1 |
| Résistance à la rupture (en newton) | 85,3 | 97,0 | 91,2 | 59,8 | 130,4 |
| Friabilité (exprimée en %) | ** | 45 | 45 | 81 | 70 |
| Ecoulement (indice de Carr) | 83 | 85 | 85 | 82 | 85 |

* granulométrie moyenne, correspond à 50 % de la distribution en masse
** non mesurable d'après le test indiqué

0 179 703

Toutes ces valeurs sont obtenues pour une granulométrie moyenne plus basse que celle du mannitol directement compressible connu, avec ou sant liant, ce qui destine le mannitol conforme à l'invention à la fabrication de comprimés pharmaceutiques faiblement dosés en principes actifs.

Comme il va de soi et comme il résulte d'ailleurs déjà de ce qui précède, l'invention ne se limite nullement à ceux de ses modes d'application et de réalisation qui ont été plus particulièrement envisagés ; elle en embrasse, au contraire, toutes les variantes.

**Revendications**

1. Mannitol granulaire directement compressible et exempt de liant, caractérisé par le fait que sa fraction granulométrique de 400 à 500 μm présente une friabilité inférieure à 65 % dans un test A, indiqué dans la description.

2. Mannitol granulaire directement compressible et exempt de liant selon la revendication 1, caractérisé par le fait qu'il présente une compressibilité supérieure à 49,0 N, de préférence à 78,4 N dans un test B, indiqué dans la description.

3. Mannitol granulaire directement compressible et exempt de liant selon l'une des revendications 1 et 2, caractérisé par le fait qu'il présente un indice d'écoulement supérieur à 65 dans un test C, indiqué dans la description.

4. Mannitol granulaire directement compressible et exempt de liant selon l'une quelconque des revendications 1 à 3, caractérisé par le fait qu'il présente une granulométrie moyenne (taille moyenne correspondant à 50 % de la distribution en masse) inférieure à 650 μm favorisant son utilisation dans la préparation de comprimés pharmaceutiques faiblement dosés en principes actifs.

5. Procédé de fabrication de mannitol granulaire directement compressible et exempt de liant, d'une friabilité inférieure à 65 % dans un test A, caractérisé par le fait que l'on introduit simultanément et en continu dans un récipient ouvert, d'axe horizontal ou incliné sur l'horizontale, entraîné en rotation autour dudit axe à une vitesse de 2 à 10 tours/minute, contenant une masse de granules de mannitol d'une température de 145 à 165 °C, maintenue en mouvement par la rotation du récipient,

— d'une part, du mannitol cristallisé d'une granulométrie inférieure à 5 mm, de préférence inférieure à 1,5 mm, et d'une température de 5 à 150 °C, de préférence de 50 à 150 °C,

— d'autre part, du mannitol essentiellement à l'état liquide, divisé ou non en gouttelettes d'une teneur en matières sèches supérieure à 99 % et d'une température comprise entre 164 et 180 °C et, de préférence de 166,5 à 180 °C, et plus avantageusement encore de 175 à 180 °C, le mannitol cristallisé et le mannitol liquide étant amenés à la surface de la masse de granules en mouvement suivant un rapport de 6 à 1,5 parties en poids de mannitol liquide pour 1 partie en poids de mannitol cristallisé, ces paramètres étant sélectionnés à l'intérieur des limites indiquées de telle sorte qu'il ne se produise pas de fusion totale à la surface de la masse en mouvement où se rejoignent le mannitol essentiellement à l'état liquide et le mannitol cristallisé, les granules de grande taille qui ont tendance à monter à la surface de la masse en mouvement par un phénomène de ségrégation, étant recueillis de préférence par débordement vers l'extérieur du récipient rotatif.

6. Procédé selon la revendication 5, caractérisé par le fait que les granules recueillis à la sortie du récipient 1 sont soumis à une phase de mûrissement par maintien à une température de 70 à 80 °C pendant 5 à 60 minutes.

7. Mannitol granulaire directement compressible caractérisé par le fait qu'il a été préparé par mise en œuvre du procédé selon les revendications 5 et 6.

8. Mannitol granulaire directement compressible susceptible d'être obtenu par le procédé selon les revendications 5 et 6.

**Claims**

1. Directly compressible granular mannitol free from binder, characterized by the fact that its granulometric fraction of 400 to 500 μm has a friability less than 65 % in a test A, indicated in the description.

2. Directly compressible granular mannitol free from binder according to Claim 1, characterized by the fact that it has a compressibility higher than 49.0 N, preferably than 78.4 N in a test B, indicated in the description.

3. Directly compressible granular mannitol free from binder according to one of claims 1 and 2, characterized by the fact that it has a flow index higher than 65 in a test C, indicated in the description.

4. Directly compressible granular mannitol free from binder according to any one of Claims 1 to 3, characterized by the fact that it has an average granulometry (average size corresponding to 50 % of the distribution by weight) less than 650 μm facilitating its use in the preparation of pharmaceutical tablets weakly dosed with active principles.

5. Method to prepare the directly compressible and binderless granular mannitol, of friability less than 65 % in a test A, characterized by the fact that there is introduced simultaneously and continuously

7

into an open vessel, whose axis is horizontal or inclined with respect to the horizontal, which is rotated around said axis at a speed of 2 to 10 rpm and which contains a mass of mannitol granules having a temperature of 145 to 165 °C and kept in motion by the rotation of the vessel,
— on the one hand, crystalline mannitol of granulometry less than 5 mm, preferably less than 1.5 mm, and of a temperature from 5 to 150 °C, preferably from 50 to 150 °C,
— on the other hand, mannitol essentially in the liquid state, divided or not into droplets of dry matter content higher than 99 % and of a temperature comprised between 164 and 180 °C and, preferably from 166.5 to 180 °C, and more advantageously still from 175 to 180 °C,
the crystalline mannitol and the liquid mannitol being brought to the surface of the mass of granules in motion in a ratio of 6 to 1.5 parts by weight of liquid mannitol per 1 part by weight of crystalline mannitol, these parameters being selected within the limits indicated so that total fusion at the surface of the mass in motion does not occur where the mannitol essentially in the liquid state and the crystalline mannitol meet, the granules of large size which have a tendancy to rise to the surface of the mass in motion by a segregation phenomenon being collected preferably by overflow to the outside of the rotary vessel.

6. Method according to Claim 5, characterized by the fact that the granules collected at the exit from the vessel 1 are subjected to a ripening phase by maintaining at a temperature of 70 to 80 °C for 5 to 60 minutes.

7. Directly compressible mannitol characterized by the fact that it has been prepared by the process according to Claims 5 and 6.

8. Directly compressible mannitol liable to be prepared by the process according to Claims 5 and 6.

## Patentansprüche

1. Direkt komprimierbarer und von Bindemittel freier körniger Mannit, dadurch gekennzeichnet, daß seine Korngrößenfraktion von 400 bis 500 μm in einem in der Beschreibung angegebenen Test A eine geringere Zerreibbarkeit als 65 % aufweist.

2. Direkt komprimierbarer und von Bindemittel freier körniger Mannit nach Anspruch 1, dadurch gekennzeichnet, daß er in einem in der Beschreibung angegebenen Test B eine höhere Komprimierbarkeit als 49,0 N, vorzugsweise als 78,4 N, aufweist.

3. Direkt komprimierbarer und von Bindemittel freier körniger Mannit nach Anspruch 1 oder 2, dadurch gekennzeichnet, daß er in einem in der Beschreibung angegebenen Test C einen Fließfähigkeitsindex von größer als 65 aufweist.

4. Direkt komprimierbarer und von Bindemittel freier körniger Mannit nach einem der Ansprüche 1 bis 3, dadurch gekennzeichnet, daß er eine mittlere Korngröße (mittlere Größe, die 50 % der Massenverteilung entspricht) von weniger als 650 μm aufweist, was seine Verwendung zur Herstellung von an Wirkstoffen schwach dosierten pharmazeutischen Tabletten begünstigt.

5. Verfahren zur Herstellung von direkt komprimierbarem und von Bindemittel freiem körnigem Mannit, der eine geringere Zerreibbarkeit als 65 % in einem Test A aufweist, dadurch gekennzeichnet, daß man gleichzeitig und kontinuierlich in einen offenen Behälter, dessen Achse horizontal oder zur Horizontale geneigt ist, der um die genannte Achse mit einer Geschwindigkeit von 2 bis 10 Umdrehungen pro Minute angetrieben wird, und der eine auf einer Temperatur von 145 bis 165 °C gehaltene Masse von Mannitkörnchen enthält, welche Masse durch die Drehung des Behälters in Bewegung gehalten wird, die folgenden Komponenten einführt :
— einerseits kristallisierten Mannit mit einer Korngröße von unter 5 mm, vorzugsweise unter 1,5 mm, und von einer Temperatur von 5 bis 150 °C, vorzugsweise 50 bis 150 °C,
— anderseits Mannit, der sich im wesentlichen im flüssigen Zustand befindet, und der auf Tröpfchen mit einem Gehalt an Trockensubstanz von mehr als 99 % und mit einer Temperatur, die zwischen 164 und 180 °C liegt, vorzugsweise zwischen 166,5 und 180 °C liegt, und in noch vorteilhafterem Maße zwischen 175 und 180 °C liegt, aufgeteilt ist oder nicht,
wobei der kristallisierte Mannit und der flüssige Mannit auf die Oberfläche der in Bewegung befindlichen Körnchenmasse in einem Verhältnis von 6 bis 1,5 Gewichtsteilen an flüssigem Mannit auf 1 Gewichtsteil an kristallisiertem Mannit aufgebracht werden, und wobei diese Parameter innerhalb der angegebenen Grenzen derart ausgewählt werden, daß auf der Oberfläche der in Bewegung befindlichen Masse, auf welcher Oberfläche der im wesentlichen im flüssigen Zustand befindliche Mannit und der kristallisierte Mannit zusammenkommen, kein vollständiges Schmelzen stattfindet, und wobei die Körnchen mit großer Korngröße, die die Tendenz zeigen, auf Grund eines Segregationsphänomens zur Oberfläche der in Bewegung befindlichen Masse aufzusteigen, vorzugsweise durch Überfließen zur Außenseite des Drehbehälters abgetrennt werden.

6. Verfahren nach Anspruch 5, dadurch gekennzeichnet, daß die am Ausgang des Behälters 1 abgetrennten Körnchen einer Reifungsphase dadurch unterworfen werden, daß man sie während 5 bis 60 Minuten auf einer Temperatur von 70 bis 80 °C hält.

7. Direkt komprimierbarer körniger Mannit, dadurch gekennzeichnet, daß er durch Anwendung des Verfahrens nach den Ansprüchen 5 und 6 hergestellt worden ist.

8. Direkt komprimierbarer körniger Mannit, erhältlich nach dem Verfahren nach den Ansprüchen 5 und 6.

# Fig.1.

0,75

3,5

4,4

5,0

45°

0,75

ø 19

ø 20

Fig.2.

0 179 703